# EUROPEAN PATENT APPLICATION

(11) **EP 4 201 425 A1**
(43) Date of publication of application: **28.06.2023**
(21) Application number: 21858367.2
(22) Date of filing: 19.08.2021
(51) Int. Cl.: A61K 45/00, A61K 45/06, A61P 9/00, A61P 25/00, A61P 25/08, A61P 25/18, A61P 27/02, A61P 35/00, A61P 43/00, C07K 14/47, A61K 47/42, A61K 38/00, A61K 38/16

(54) **REVERSIBLE OPENING AGENT FOR NERVOUS SYSTEM VASCULAR BARRIER**

(30) Priority: 21.08.2020 JP 2020139886
(71) Applicant: Yamaguchi University, Yamaguchi 753-8511 (JP)
(72) Inventor: IKEDA Eiji, Ube-shi, Yamaguchi 755-8505 (JP)
(74) Representative: Hasegawa, Kan
(86) International application number: PCT/JP2021/030373
(87) International publication number: WO 2022/039223

(57) **Abstract**

It is an object of the present invention to provide a reversible opening agent for the neural vascular barrier.

As a solution, the reversible opening agent for the neural vascular barrier is prepared by including a ligand having an agonistic effect on basigin as an active ingredient. It is preferable that the above ligand contains a polypeptide consisting of an amino acid sequence shown in SEQ ID NO: 1 or the like, or a salt thereof as the active ingredient or the above ligand is cyclophilin A lacking peptidyl-prolyl cis-trans isomerase activity.

## Description

### TECHNICAL FIELD

The present invention relates to an agent that reversibly opens the neural vascular barrier.

### BACKGROUND ART

In multicellular organisms including humans, adult neural tissues such as the brain and the retina are separated from other tissues by barriers formed by tissue-specifically differentiated blood vessels such as the blood-brain barrier (BBB) and the blood-retinal barrier (BRB) (hereinafter, also referred to as "neural vascular barrier"), thereby strongly restricting transport of substances between the blood and the neural tissue parenchyma. Such a neural vascular barrier is induced during ontogeny and is fundamentally in a closed state in adults. The barrier function is carefully regulated to maintain the optimal tissue microenvironment, which is required for nerve cells to function properly. Thus, the neural vascular barrier is extremely important for the functions of the neural tissues. On the other hand, it also prevents a therapeutic agent for neurological disease or the like administered systemically from reaching the neural tissue parenchyma with a lesion, making it more difficult to treat the disease.

Development of new therapeutic agents (drug discovery) is underway for many intractable neurological diseases for which there are still no effective treatment methods. One of the biggest obstacles in the drug discovery process is the existence of the neural vascular barrier. Many attempts have been made to cause drugs to pass through the neural vascular barrier and reach the neural tissue parenchyma. One method involves administering a hypertonic solution of mannitol into the blood vessel in order to deform the cerebral vascular endothelial cells by dehydration and physically dissociate the endothelial cells from each other, followed by administration of the therapeutic agent of interest. However, this method causes cell damages and is not an effective method. Further, regarding the neural vascular barrier, a method of opening the neural vascular barrier with a proteinase such as a matrix metalloproteinase is disclosed (refer to Non-Patent Literature 1). However, this method destroys neural vascular barrier-forming molecules, making it difficult to transiently and reversibly open the neural vascular barrier.

Despite many studies to date, no effective method has been established to cause a drug to pass through the neural vascular barrier and reach the neural tissue parenchyma. Thus, if a drug or method for artificially opening the closed neural vascular barrier with fewer side effects is developed, the major obstacle to drug discovery for the neurological diseases is removed, leading to the establishment of effective treatment methods for achieving improved prognosis and complete recovery of patients. Further, the drug that opens the neural vascular barrier can be generally applied to many intractable neurological diseases, making an extremely wide range of contribution to clinical medicine.

The present inventors began to analyze the control mechanism of the neural vascular barrier by first focusing on the mechanism in which the neural vascular barrier is opened by a hypoxic stimulation. As a result, the present inventors have found and reported a cascade in which the hypoxic stimulation causes disappearance of claudin-5 from the cell membranes of vascular endothelial cells, resulting in opening of the neural vascular barrier (refer to Non-Patent Literature 2). After further analysis, the present inventors have identified a disintegrin and metalloproteinase (ADAM)12, ADAM17, and basigin, which are cell membrane molecules expressed in the vascular endothelial cells that form the neural vascular barrier, as molecules involved in the disappearance of claudin-5 from the cell membranes of the vascular endothelial cells caused by the hypoxic stimulation. Further, the present inventors have reported that ADAM12 and ADAM17 are molecules that act relatively specifically in the process of opening the neural vascular barrier caused by the hypoxic stimulation, whereas basigin is a molecule that acts more generally in the process of opening the neural vascular barrier caused not only by the hypoxic stimulation but also by various stimuli such as an inflammatory stimulation (refer to Patent Literature 1 and Non-Patent Literatures 3 and 4).

Cyclophilin A (CypA) is a substance identified as an intracellular molecule that binds to cyclosporin A, a drug that suppresses activation of T lymphocytes. Subsequent studies have reported that CypA is also expressed in cell types other than T lymphocytes, CypA is a molecule which is also secreted extracellularly for functioning, and CypA is secreted extracellularly and binds to basigin. Further, CypA, which acts extracellularly, has been shown to be involved in several biological phenomena such as modification in an inflammatory process.

It has been reported that such cyclophilin A is conjugated to a compound that facilitates transport across the blood-brain barrier, such as a transferrin receptor-binding antibody, and used as a neuroprotective agent (refer to Patent Literature 2), a cyclophilin A/MMP9 pathway is involved in degradation of blood-brain barrier constituent molecules caused by apoE4 (refer to Non-Patent Literature 5), and cyclophilin A and basigin (CD147) are involved in apoptosis during subarachnoid hemorrhage (refer to Non-Patent Literature 6). However, it has not been known until now that cyclophilin A reversibly opens the neural vascular barrier.

### CITATION LIST

### PATENT LITERATURE

Patent Literature 1: WO 2017/073232
Patent Literature 2: JP 2008-530029 T

### NON PATENT LITERATURE

Non Patent Literature 1: Neuroscience Letters Volume 649, 10 Pages 7-13, 2017
Non Patent Literature 2: Am. J. Pathol. Volume 170, Issue 4, Pages 1389-1397, 2007
Non Patent Literature 3: Scientific Report 5, 12796; doi: 10.1038/srep12796, 2015
Non Patent Literature 4: Scientific Report 6, 38445; doi: 10.1038/srep38445, 2016
Non Patent Literature 5: JAMA Neurol. September 1; 70(9): 1198-1200; doi:10.1001/jamaneurol.2013.3841
Non Patent Literature 6: Critical Care Medicine, September 2015, Volume 43; Number 9 e369-381

### SUMMARY OF INVENTION

### TECHNICAL PROBLEM

It is an object of the present invention to provide a reversible opening agent for the neural vascular barrier.

### SOLUTION TO PROBLEM

As a result of intensive studies to solve the above-mentioned problems, the present inventors have first found that stimulation of the cerebral microvascular endothelial cells by cyclophilin A (CypA), a known agonist of basigin, results in loss of neural vascular barrier properties in the cerebral microvascular endothelial cells in an in vitro system analysis and that such a loss of the neural vascular barrier properties is transient and reversible. Further, the present inventors have found that, in an in vivo system analysis, injection of CypA into mice transiently and reversibly opens the neural vascular barrier in vivo and that pre-injection of CypA makes it possible to actually deliver systemically administered doxorubicin, a drug with low intracerebral penetration, into the mouse neural tissue, thereby completing the present invention.

That is, the present invention is as follows.
[1] A reversible opening agent for a neural vascular barrier, including a ligand having an agonistic effect on basigin as an active ingredient.
[2] The reversible opening agent for the neural vascular barrier according to the above [1] characterized in that the ligand is a polypeptide ligand.
[3] The reversible opening agent for the neural vascular barrier according to the above [2] characterized in that the polypeptide ligand contains as the active ingredient one or more polypeptides or salts thereof selected from the group consisting of:
   (1) a polypeptide consisting of an amino acid sequence shown in SEQ ID NO: 1;
   (2) a polypeptide consisting of an amino acid sequence in which one or several amino acids are added, substituted, deleted, and/or inserted in the amino acid sequence shown in SEQ ID NO: 1, and having a reversible opening effect on the neural vascular barrier; and
   (3) a polypeptide consisting of an amino acid sequence having at least 80% or more identity with the amino acid sequence shown in SEQ ID NO: 1, and having the reversible opening effect on the neural vascular barrier.
[4] The reversible opening agent for the neural vascular barrier according to the above [2] or [3] characterized in that the polypeptide ligand is cyclophilin A lacking peptidyl-prolyl cis-trans isomerase activity.
[5] The reversible opening agent for the neural vascular barrier according to any of the above [1] to [4], the agent being administered simultaneously or successively with at least one therapeutic agent selected from a therapeutic agent for neurodegenerative disease, a therapeutic agent for retinal disease, a therapeutic agent for psychiatric disorder, a therapeutic agent for central neural system tumor, and a therapeutic agent for epilepsy.

Further, other aspects of the present invention are as follows.
<1> A method for delivering at least one therapeutic agent selected from a therapeutic agent for neurodegenerative disease, a therapeutic agent for retinal disease, a therapeutic agent for psychiatric disorder, a therapeutic agent for central neural system tumor, and a therapeutic agent for epilepsy to the brain of a subject, the method including a step of administering simultaneously or successively a reversible opening agent for the neural vascular barrier containing a ligand having an agonistic effect on basigin as an active ingredient, and at least one therapeutic agent selected from a therapeutic agent for neurodegenerative disease, a therapeutic agent for retinal disease, a therapeutic agent for psychiatric disorder, a therapeutic agent for central neural system tumor, and a therapeutic agent for epilepsy to the subject.
<2> A use of the ligand having the agonistic effect on basigin for producing the reversible opening agent for the neural vascular barrier.

### ADVANTAGEOUS EFFECTS OF INVENTION

Using the reversible opening agent for the neural vascular barrier of the present invention makes it possible to reversibly open the neural vascular barrier.

### BRIEF DESCRIPTION OF DRAWINGS

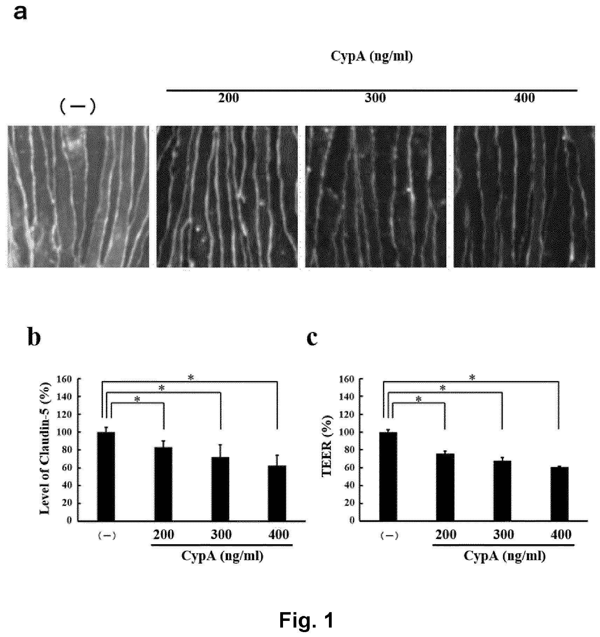

FIG. 1 is a diagram showing results of treatment with CypA in an amount of 200, 300, or 400 ng/ml in Example 1. FIG. 1a is a diagram showing a result of immunofluorescence staining, FIG. 1b is a diagram showing a result of quantification of claudin-5 signals on the cell membranes, and FIG. 1c is a diagram showing a measurement result of transepithelial electrical resistance (TEER) of the cell monolayer.

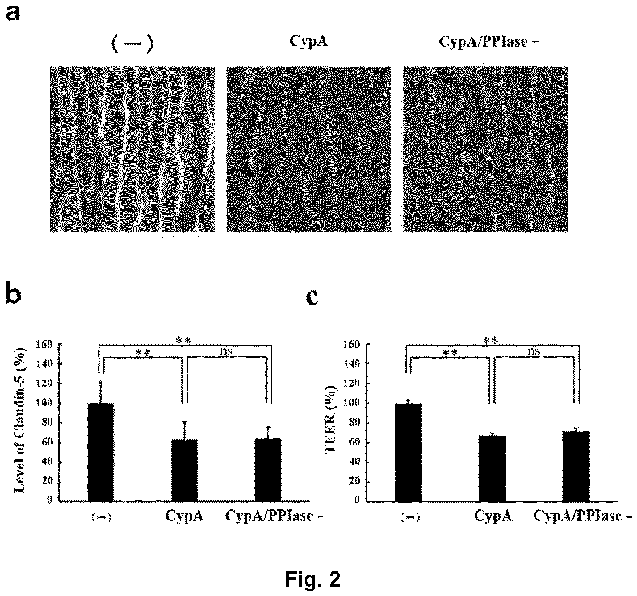

FIG. 2 is a diagram showing results of treatment with CypA in an amount of 300 ng/ml with PPIase activity (CypA in the drawing) or without PPIase activity (CypA/PPIase- in the drawing) in Example 2. FIG. 2a is a diagram showing a result of immunofluorescence staining, FIG. 2b is a diagram showing a result of quantification of claudin-5 signals on the cell membranes, and FIG. 2c is a diagram showing a measurement result of TEER of the cell monolayer.

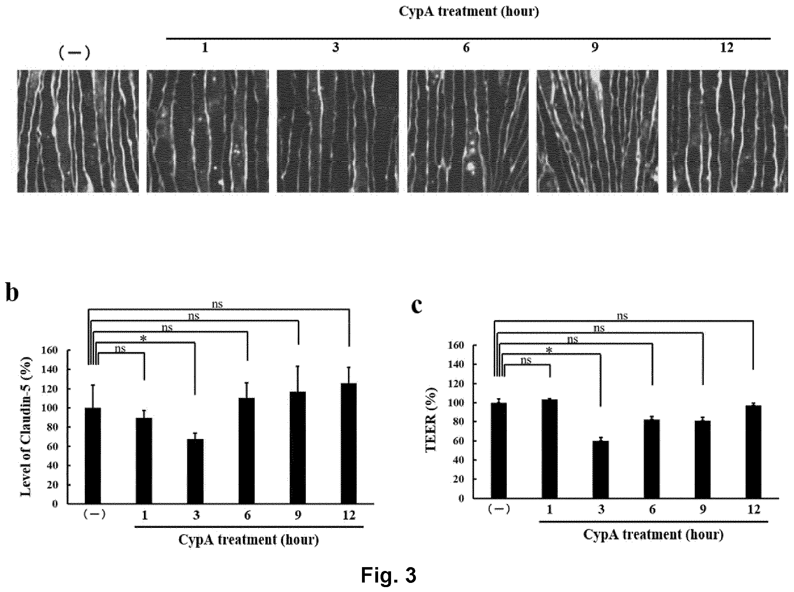

FIG. 3 is a diagram showing results of treatment with CypA in an amount of 300 ng/ml over time in Example 3. FIG. 3a is a diagram showing a result of immunofluorescence staining, FIG. 3b is a diagram showing a result of quantification of claudin-5 signals on the cell membranes, and FIG. 3c is a diagram showing a measurement result of TEER of the cell monolayer.

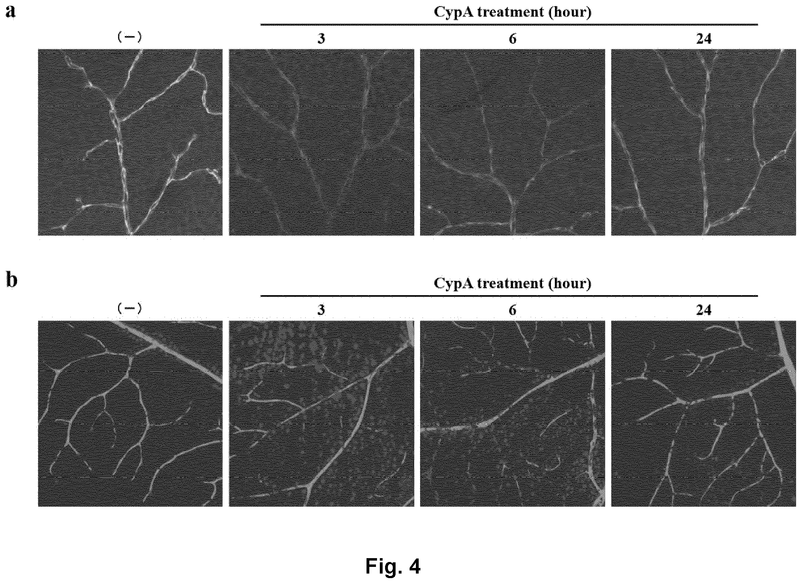

FIG. 4 is a diagram showing results of examining a change in barrier function by administration of CypA using the mouse retinal tissues in Example 4. FIG. 4a is a diagram showing a result of immunofluorescence staining and FIG. 4b is a diagram showing a result of leakage of an intravenously injected tracer dye.

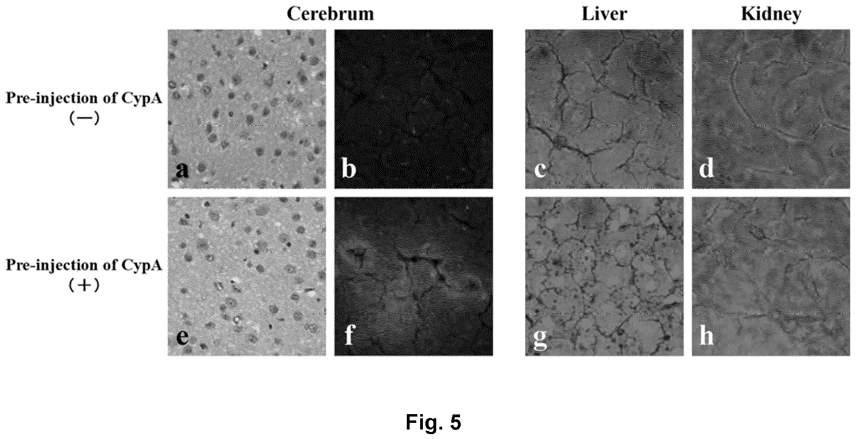

FIG. 5 is a diagram showing results in which doxorubicin is administered intravenously to mice with or without preadministration of CypA and uptake of the doxorubicin into the cerebrum, the liver, and the kidney is observed using a laser confocal microscope in Example 5. An upper part shows the result without preadministration of CypA and a lower part shows the result with preadministration of CypA. FIGS. 5a and 5e show HE staining of the cerebrum, and FIGS. 5b, 5c, 5d, 5f, 5g, and 5h show doxorubicin fluorescence in the cerebrum, the liver, and the kidney.

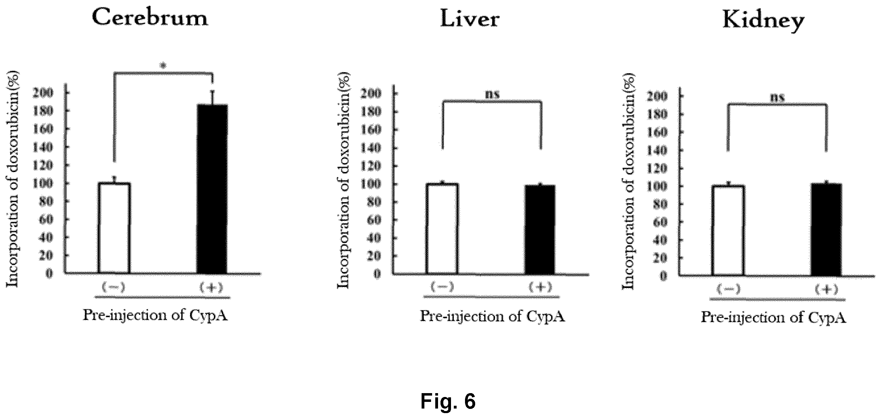

FIG. 6 is a diagram showing results in which doxorubicin is administered intravenously to mice with or without preadministration of CypA and uptake of the doxorubicin into the cerebrum, the liver and the kidney is quantified in Example 5.

### DESCRIPTION OF EMBODIMENTS

Basigin in the present specification is a glycoprotein belonging to the immunoglobulin superfamily that is localized in the cell membrane. It is also called EMMPRIN (extracellular matrix metalloproteinase inducer), CD147 (cluster of differentiation 147), HT7, OX-47, or forth22. Basigin is known to be a receptor for cyclophilin A (CypA), a member of cyclophilins with peptidyl-prolyl cis-trans isomerase (PPIase) activity. Basigin is preferably derived from human. Examples of human basigin include SEQ ID NOS: 2 to 4, which are publicly available under accession numbers NP_001719.2, NP_940991.1, and NP_940991.1, respectively, in the National Center for Biotechnology Information (NCBI).

As a ligand having an agonistic effect on basigin in the present specification is not particularly limited as long as it is a compound that binds to basigin and has an effect of reversibly opening the neural vascular barrier. Such a ligand may be any of a polypeptide, an antibody, a protein, a nucleic acid, and a low molecular weight compound. However, the ligand is preferably a polypeptide.

As such a polypeptide ligand, the following polypeptides or salts thereof can be more preferably mentioned.

One or more peptides selected from the group consisting of:
(1) a peptide consisting of an amino acid sequence shown in SEQ ID NO: 1;
(2) a peptide consisting of an amino acid sequence in which one or several amino acids are added, substituted, deleted, and/or inserted in the amino acid sequence shown in SEQ ID NO: 1, and having a reversible opening effect on the neural vascular barrier; and
(3) a peptide consisting of an amino acid sequence having at least 80% or more identity with the amino acid sequence shown in SEQ ID NO: 1, and having the reversible opening effect on the neural vascular barrier.

Further, cyclophilin A (CypA), which is a member of cyclophilins, can also be mentioned as the above-mentioned polypeptide ligand. Cyclophilin A (CypA) is a substance identified as an intracellular molecule that binds to cyclosporin A, a drug that suppresses activation of T lymphocytes. The amino acid sequence of such cyclosporin is available from a website of the above-mentioned NCBI and published under accession number NP_066953.1 (SEQ ID NO: 1). Further, cyclophilin A (CypA) may be with or without peptidyl-prolyl cis-trans isomerase (PPIase) activity. However, CypA without the PPIase activity is preferable from the viewpoint of having fewer side effects.

The above-mentioned "amino acid sequence in which one or several amino acids are added, substituted, deleted, and/or inserted" means an amino acid sequence having addition, substitution, deletion, and/or insertion of any number of amino acids, which is, for example, 1 to 20, preferably 1 to 10, more preferably 1 to 3, still more preferably 1 or 2, most preferably 1. The above-mentioned "amino acid sequence having at least 80% or more identity with the amino acid sequence shown in SEQ ID NO: 1" means that the sequence identity with the amino acid sequence shown in SEQ ID NO: 1 is 80% or more, preferably 90% or more, more preferably 95% or more, still more preferably 98% or more.

The above-mentioned polypeptide may be a naturally occurring or modified polypeptide. Examples of the modified polypeptide include a D-peptide or L-peptide; an α-peptide, β-peptide, or γ-peptide; an N-methyl peptide; an azapeptide; a polypeptide having one or more amide (i.e., peptide) bonds substituted with one or more urea, thiourea, carbamate, or sulfonylurea bonds, and a polypeptide modified by addition of a biochemical functional group. Further, the above-mentioned polypeptide may have a carboxyl group (-COOH), carboxylate (-COO-), amide (-CONH₂), or ester (-COOR) at the C-terminus. Examples of R in the ester include a C1-6 alkyl group such as methyl, ethyl, n-propyl, isopropyl, or n-butyl; a C3-8 cycloalkyl group such as cyclopentyl or cyclohexyl; a C6-12 aryl group such as phenyl or α-naphthyl, and a phenyl-C1-2 alkyl group such as benzyl or phenethyl.

The salts in the "polypeptides or salts thereof" in the present specification are not particularly limited as long as they are pharmacologically acceptable salts. Specific examples thereof include an inorganic salt such as a hydrochloride, a sulfate, or a phosphate; an organic acid salt such as an acetate or a citrate; an alkali metal salt such as a sodium salt or a potassium salt; and an alkaline earth metal salt such as a magnesium salt or a calcium salt.

The above-mentioned CypA may be obtained as a commercial product, produced by a known genetic engineering technique based on the nucleotide sequence encoding CypA, or produced by a known amino acid synthesis technique based on information on the amino acid sequence of CypA. A method for producing the above-mentioned polypeptide is not particularly limited, and it can be produced according to a known peptide synthesis method based on the amino acid sequence information of the above-mentioned polypeptide. As the peptide synthesis method, for example, either a solid phase synthesis method or a liquid phase synthesis method may be used. In a case of producing the above-mentioned polypeptide using the solid-phase synthesis method, a peptide or an amino acid that can constitute the above-mentioned polypeptide is condensed with the remaining portion of the polypeptide by using the solid-phase synthesis method such as, for example, the Fmoc method (fluorenylmethyloxycarbonyl method) or the tBoc method (t-butyloxycarbonyl method), and then (if the resultant product has a protecting group) the protecting group is removed, so that the above-mentioned polypeptide of interest can be produced. For the solid-phase synthesis method, a commercially available peptide synthesizer such as APEX 396 (manufactured by Advanced ChemTech), 433A (manufactured by Applied Biosystems), PS3 (manufactured by Protein Technologies), 9050 (manufactured by PerSeptive Biosystems), or PSSM-8 (manufactured by Shimadzu Corp.) can be used. A resin used in the solid-phase synthesis method is not particularly limited, and examples thereof include "Rink amide AM Resin", "Fmoc-AA-Wang Resin", and "AA-2-Cl-Trt Resin". Further, in a case of producing the above-mentioned polypeptide using the liquid phase synthesis method, the above-mentioned polypeptide can be produced by a method of stepwise condensation of N-protected amino acid derivatives one residue at a time. A method such as the dicyclohexylcarbodiimide (DCC) method, the active ester method, or the mixed acid anhydride method can be used depending on the presence or absence of the protecting group. Further, the above-mentioned polypeptide can also be produced by a method using a condensing agent such as 1-ethyl-3-(3-dimethylaminopropyl)carbodiimide hydrochloride (WSC-HCl), a (1H-benzotriazol-1-yloxy)tris(dimethylamino)phosphonium hexafluorophosphate (BOP) reagent, or diisopropylcarbodiimide (DIPCDI). Further, a commercially available product can be purchased and used.

The "neural vascular barrier" described in the present specification means a mechanism that restricts substance exchange between the blood and the tissue fluid of the neural system, and preferable examples thereof include the blood-brain barrier, which restricts substance exchange between the blood and the cerebral tissue fluid and the blood-retinal barrier, which restricts substance exchange between the blood and the retinal tissue fluid. Note that the term "neural vascular" refers to blood vessels of the neural tissues and does not include blood vessels in tissues other than the neural tissues.

In the present specification, the term "reversible opening of the neural vascular barrier" means that the above-mentioned mechanism that restricts substance exchange between the blood and the tissue fluid of the neural system becomes not functional or less functional, thereby making it possible to cause transient and reversible substance exchange between the blood and the tissue fluid of the neural system. The term "reversible opening" described herein means that the neural vascular barrier does not remain open but can recover to a state in which the substance exchange between the blood and the tissue fluid of the neural system is restricted after a certain period of time.

In the present specification, the term "transiently" means that elapsed time after administration of the reversible opening agent for the neural vascular barrier of the present invention to a subject is, for example, in a range of 0.2 to 24 hours with a lower limit of 0.5 hours, 1 hour, 2 hours, 3 hours, 4 hours, 5 hours, or 6 hours and with an upper limit of 20 hours, 18 hours, 16 hours, 12 hours, 10 hours, or 9 hours.

The reversible opening agent for the neural vascular barrier of the present invention may be administered simultaneously or successively with at least one therapeutic agent selected from a therapeutic agent for neurodegenerative disease, a therapeutic agent for retinal disease, a therapeutic agent for psychiatric disorder, a therapeutic agent for central neural system tumor, and a therapeutic agent for epilepsy. The term "administered simultaneously" described herein means administration of two or more agents to the same subject at the same time. Further, the term "administered successively" means that two or more agents are administered to the same subject successively, that is, administered sequentially or separately at certain intervals.

In one embodiment according to the present invention, the reversible opening of the neural vascular barrier by the reversible opening agent lasts for 12 hours, 11 hours, 10 hours, 9 hours, 8 hours, 6 hours, 4 hours, 3 hours, 2 hours, 1 hour, 30 minutes, 15 minutes, 10 minutes, or 5 minutes.

When the agents are administered sequentially or separately at certain intervals, the "certain interval" is, for example, in a range of 0.5 minutes to 10 hours with a lower limit of, for example, 1 minute, 2 minutes, 3 minutes, 5 minutes, 10 minutes, 15 minutes, 30 minutes, 1 hour, 2 hours, or 3 hours and with an upper limit of 8 hours, 6 hours, 5 hours, 4 hours, or 3 hours.

The opening agent for the neural vascular barrier of the present invention is only required to include a ligand having the agonistic effect on basigin as an active ingredient. The opening agent may further include a constituent ingredient as needed, and examples thereof include a conventional pharmaceutically acceptable carrier, binder, stabilizer, excipient, diluent, pH buffer, disintegrant, isotonic agent, additive, coating agent, solubilizer, lubricating agent, sliding agent, solubilizing aid, lubricant, flavoring agent, sweetener, solvent, gelling agent, and nutrient. Specific examples of such a constituent ingredient include water, physiological saline, animal fat and oil, vegetable oil, lactose, starch, gelatin, crystalline cellulose, gum, talc, magnesium stearate, hydroxypropyl cellulose, polyalkylene glycol, polyvinyl alcohol, and glycerin.

An administration method of the opening agent for the neural vascular barrier of the present invention is not particularly limited as long as the desired opening effect of the neural vascular barrier of the present invention is obtained. Examples of the administration method include intravenous administration, oral administration, intravitreal administration, intramuscular administration, subcutaneous administration, transdermal administration, nasal administration, and transpulmonary administration. Further, a dose of the opening agent for the neural vascular barrier of the present invention is not particularly limited and can be appropriately adjusted depending on a physical condition, a medical condition, weight, age, sex, and the like of a human subject or an animal subject. The dose may be, for example, 0.01 µg to 100 g/kg body weight, more preferably 0.1 µg to 10 g/kg body weight, and still more preferably 1 µg to 1 g/kg body weight per day, per day. The administration may be performed once daily or multiple times in divided doses (e.g., 2 to 4 times).

The therapeutic agent is not particularly limited as long as it is at least one therapeutic agent selected from a therapeutic agent for neurodegenerative disease, a therapeutic agent for retinal disease, a therapeutic agent for psychiatric disorder, a therapeutic agent for central neural system tumor, and a therapeutic agent for epilepsy. Examples of the therapeutic agent for neurodegenerative disease include therapeutic agents for Alzheimer's disease, Parkinson's disease, Huntington's disease, amyotrophic lateral sclerosis, spinocerebellar degeneration, multiple sclerosis, myasthenia gravis, cerebral infarction, vascular dementia, and the like. Examples of the therapeutic agent for retinal disease include therapeutic agents for diabetic retinopathy, age-related macular degeneration, retinal edema, retinal detachment, proliferative vitreoretinopathy, uveitis, eye infection, retinopathy of prematurity, neovascular maculopathy, chorioretinopathy, and the like. Examples of the therapeutic agent for psychiatric disorder include therapeutic agents for depression, schizophrenia, panic disorder, and the like. Examples of the therapeutic agent for central neural system tumor include therapeutic agents for glioma, central neural system lymphoma, glioblastoma multiforme, gliosarcoma, and the like. Examples of such therapeutic agents include a low molecular weight compound, an antisense oligonucleotide, a ribozyme or a protein, and a polypeptide or a peptide.

A subject to which the opening agent for the neural vascular barrier of the present invention is administered is not particularly limited. Examples of the subject include a mammal such as a human, a monkey, a cow, a horse, a sheep, a pig, a dog, a cat, a rat, a mouse, or a hamster.

Hereinafter, the present invention will be described in more detail below by way of examples. However, the technical scope of the present invention is not limited to these examples.

### EXAMPLES

### [Example 1] (In vitro system analysis using monolayer culture of mouse cerebrovascular endothelial cell line (1))

As described above, the present inventors have previously found and reported the cascade in which "the vascular barrier is opened by disappearance of claudin-5 from the cell membranes in the vascular endothelial cells". Thus, an expression level of claudin-5 by administration of CypA was examined.

In an in vitro system, mouse brain microvascular endothelial cell line bEnd.3 cells (obtained from the American Type Culture Collection) were grown as a monolayer at 37°C under 5% CO² in Dulbecco's modified Eagle's medium containing 4500 mg/L glucose (Sigma-Aldrich) supplemented with 10% fetal bovine serum (FBS). CypA (200, 300 or 400 ng/ml: BioVendor R&D) was added to the culture supernatant of the monolayer-cultured bEnd.3 cells, followed by incubation for 3 hours. Subsequently, expression changes of claudin-5 in the bEnd.3 cells were observed by immunofluorescence staining, and claudin-5 signals on the cell membranes were quantified. Further, TEER, an index of vascular barrier function, was measured in the bEnd.3 cell monolayer.

The immunofluorescence staining was performed as follows. First, the cultured bEnd.3 cells were fixed with 100% methanol for 5 min at room temperature and incubated with 10% non-immune goat serum (Invitrogen) for 30 minutes to block non-specific binding of antibodies. Next, the cells were reacted with rabbit polyclonal antibodies against claudin-5 (1/25 dilution, Invitrogen) at 4°C overnight. After washing with phosphate-buffered saline (PBS), the cells were incubated with Alexa Fluor 488 goat anti-rabbit IgG (1/200 dilution, Eugene) for 1 hour at room temperature under light protection. Then, the stained cells were mounted in Fluoromount (Diagnostic BioSystems Inc.) and observed under a Zeiss LSM5 Pascal laser confocal microscope (Carl Zeiss AG). For a quantitative analysis, fluorescence intensities of claudin-5 on the plasma membranes were measured using an operation menu installed in LSM5 Pascal6 ,7.3. Fields in the culture dish were randomly photographed and 5 straight lines were drawn on each photograph. Fluorescence intensities were then quantified at points on the cell membranes that intersected the drawn straight lines. The mean value of the fluorescence intensities was calculated as an expression level of claudin-5 on the cell membranes of each monolayer at approximately 80 points. All experiments were performed independently in triplicate.

FIG. 1a shows a result of the immunofluorescence staining, FIG. 1b shows a quantification result of the claudin-5 signals on the cell membranes, and FIG. 1c shows a measurement result of TEER of the bEnd.3 cell monolayer. FIG. 1a and FIG. 1b showed that the claudin-5 level on the cell membranes was significantly reduced 3 hours after CypA treatment. Further, FIG. 1c showed that CypA treatment correlated with the claudin-5 level on the cell membranes, and a significant reduction in the barrier function occurred 3 hours after CypA treatment. These results confirmed that a single dose of CypA stimulation opened the barrier of the bEnd.3 cell monolayer.

### [Example 2] (In vitro system analysis using monolayer culture of mouse cerebrovascular endothelial cell line (2))

CypA is known to have PPIase activity. Thus, whether or not the PPIase activity is involved in the action of CypA to open the barrier was examined by using CypA lacking the PPIase activity.

The immunofluorescence staining, the quantification of the claudin-5 signals on the cell membranes, and the measurement of TEER in the bEnd.3 cell monolayer were performed in the same manner as in Example 1 except that CypA (300 ng/ml: BioVendor R&D) lacking PPIase activity was used as CypA. The results are shown in FIG. 2a to FIG. 2c.

As is clear from FIG. 2a to FIG. 2c, even when CypA lacking PPIase activity was used, the claudin-5 signals on the cell membranes decreased similarly to when CypA having PPIase activity was used, resulting in a significant reduction in the barrier function. Thus, the opening of the neural vascular barrier by CypA was shown to be independent of the PPIase activity.

### [Example 3] (In vitro system analysis using monolayer culture of mouse cerebrovascular endothelial cell line (3))

In order to evaluate the opening effect of CypA on the neural vascular barrier over time, the same experiment as in Example 1 was performed while changing the treatment time of CypA. Specifically, CypA at a concentration of 300 ng/ml was applied to the cells, followed by incubation for 1, 3, 6, 9, and 12 hours. Then, the cells were subjected to the immunofluorescence staining, the quantification of the claudin-5 signals on the cell membranes, and the measurement of TEER in the bEnd.3 cell monolayer.

FIG. 3a shows a result of the immunofluorescence staining, FIG. 3b shows a quantification result of the claudin-5 signals on the cell membranes, and FIG. 3c shows a measurement result of TEER in the bEnd.3 cell monolayer over time. FIG. 3a and FIG. 3b showed that the claudin-5 level on the cell membranes decreased significantly 3 hours after CypA treatment but recovered to the level prior to the CypA stimulation in 6 hours. Further, FIG. 3c showed that CypA treatment was inversely correlated with the claudin-5 level on the cell membranes, and the barrier function significantly decreased 3 hours after CypA treatment and recovered to the state prior to the CypA stimulation in 6 hours. These results confirmed that a single dose of CypA stimulation transiently and reversibly opened the barrier of the bEnd.3 cell monolayer. Further, it was also confirmed that the barrier function of the opened barrier was recovered in several hours in a self-limiting manner.

### [Example 4] (In vivo system analysis using mouse retinal tissue)

Based on the results of Example 1, a change in the barrier function caused by CypA administration was examined in vivo using a mouse retinal tissue. Note that the retina is a tissue that is formed as an outgrowth of the central neural system during the process of ontogeny and is a part of the central neural system like the brain. Since the vascular system of the retina can be two-dimensionally observed and evaluated over the entire length in the longitudinal direction, the retina was used as an analysis material representing the central neural system in the present example.

Seven-week-old male C57B6/N mice (Japan SLC, Inc.) were given a single intravenous injection (200 µg/kg) of CypA. A change in the expression level of claudin-5 localized in the endothelial cell membranes of the retinal vasculature and permeability of the retinal vasculature were measured 3, 6, and 24 hours after the vitreous injection. The change in the expression of claudin-5 was measured by immunofluorescence staining using the rabbit polyclonal antibodies against claudin-5 in the same manner as in Example 1. The above-mentioned measurement of the permeability of the retinal vasculature was performed as follows.

First, 500 µL of PBS containing 100 µg/mL Hoechst dye H33258 (molecular weight 534 Da, Sigma-Aldrich) and 1 mg/mL tetramethylrhodamine-conjugated lysine-fixable dextran (molecular weight 10,000 Da, Thermo Fisher Scientific) was injected into the left ventricle as a tracer dye. After injection of the Hoechst dye, eyes were enucleated and immediately fixed with 4% paraformaldehyde (PFA) for 15 min at room temperature under light protection, thereby preparing retinal flat mounts. They were mounted in a fluorescent mounting medium, and leakage of the injected tracer was observed under a Zeiss LSM510 META laser confocal microscope (Carl Zeiss AG) to measure the permeability of the retinal vasculature (an index of blood-retinal barrier function). Experiments were performed independently 3 times or more.

As shown in FIG. 4a, the expression level of claudin-5 in the cell membranes of the peripheral microvascular endothelial cells was found to decrease 3 hours after the intravenous injection of CypA. However, the claudin-5 level recovered to a physiological level in 24 hours. Further, as shown in FIG. 4b, the leakage of the intravenously injected tracer dye increased 3 hours after the injection of CypA, decreased after 6 hours as compared with after 3 hours, and returned to an undetectable physiological level in 24 hours. These data demonstrated that a single intravenous injection of CypA caused the transient and reversible opening of the vascular barrier in the retinal vasculature.

### [Example 5] (Uptake analysis of drug into neural tissue parenchyma)

Based on the results in Examples 1 to 4 described above, it was assumed that the combined administration of CypA and a therapeutic agent could reversibly open the neural vascular barrier and allow the therapeutic agent to reach the neural tissue parenchyma. The following experiment was performed based on this assumption.

Doxorubicin was administered intravenously to mice with or without preadministration of CypA, and uptake of doxorubicin into the cerebrum, the liver, and the kidney was observed after 3 hours. Further, fluorescence emitted by doxorubicin was used to evaluate the uptake of doxorubicin into the cerebrum, the liver and the kidney.

Doxorubicin hydrochloride (6.25 mg/kg; Fujifilm Wako Pure Chemical Corp.), an anticancer drug with low intracerebral penetration, was injected into caudal veins of mice with or without pre-injection of CypA (200 µg/kg). Pre-injection of CypA was performed intravenously 3 hours prior to the injection of doxorubicin. Mice were sacrificed 3 hours after the injection of doxorubicin hydrochloride, and the cerebrum, the liver, and the kidney were collected and embedded and frozen in OCT compound. Next, frozen sections of 30 µm thickness were prepared and observed under an LSM710 laser confocal microscope (Carl Zeiss AG). Fluorescence of doxorubicin was excited with argon laser at 488 nm, and emission of light was observed through a 530-nm long-pass filter.

In FIG. 5, an upper part (a, b, c, d) shows the cerebrum, the liver, and the kidney of the mouse without preadministration of CypA. FIG. 5a shows HE staining of the cerebrum, and FIGS. 5b, 5c, and 5d show doxorubicin fluorescence of the cerebrum, the liver, and the kidney. Further, a lower part (e, f, g, h) shows the cerebrum, the liver and the kidney of the mouse with preadministration of CypA. FIG. 5e shows HE staining of the cerebrum, and FIGS. 5f, 5g, and 5h show doxorubicin fluorescence of the cerebrum, the liver, and the kidney. Further, FIG. 6 is a graph quantifying the uptake of doxorubicin in each of the cerebrum, the liver, and the kidney in FIG. 5. As is clear from FIG. 5, no doxorubicin fluorescence signal was detected in the cerebrum of the mouse without preadministration of CypA shown in the upper part. In contrast, significant doxorubicin fluorescence signals were detected in the cerebrum of the mouse with preadministration of CypA shown in the lower part. These results showed that preadministration of CypA allowed the intravenously administered doxorubicin to pass through the neural vascular barrier and reach the neural tissue parenchyma. Based on this, CypA can be used to treat neurological diseases by transiently and reversibly opening the vascular barrier of the neural tissues with little damage to the endothelial cells, allowing drugs to reach the neural tissues. Note that, in the liver and the kidney, which have the vasculature without barrier function, strong fluorescent signals of doxorubicin were detected in the mouse parenchyma with or without the pre-injection of CypA, and intensities of the fluorescence signals were not significantly affected by the pre-injection of CypA.

### INDUSTRIAL APPLICABILITY

In therapeutic strategies for intractable neurological diseases, the present invention makes it possible to deliver drugs that cannot be delivered to the neural tissues with current medical and pharmaceutical technologies to the neural tissues. In other words, the choice of drugs that can be used for the treatment of neurological diseases can be greatly expanded. Further, the present invention removes one of the major obstacles that has always stood in the way of drug discovery, that is, a difficulty in passing through the neural vascular barrier, thereby being used in medical industries.

Sequence Listing

## Claims

1. A reversible opening agent for a neural vascular barrier comprising a ligand having an agonistic effect on basigin as an active ingredient.

2. The reversible opening agent for the neural vascular barrier according to claim 1, wherein the ligand is a polypeptide ligand.

3. The reversible opening agent for the neural vascular barrier according to claim 2, wherein the polypeptide ligand contains as the active ingredient one or more polypeptides or salts thereof selected from the group consisting of:
(1) a polypeptide consisting of an amino acid sequence shown in SEQ ID NO: 1;
(2) a polypeptide consisting of an amino acid sequence in which one or several amino acids are added, substituted, deleted, and/or inserted in the amino acid sequence shown in SEQ ID NO: 1, and having a reversible opening effect on the neural vascular barrier; and
(3) a polypeptide consisting of an amino acid sequence having at least 80% or more identity with the amino acid sequence shown in SEQ ID NO: 1, and having the reversible opening effect on the neural vascular barrier.

4. The reversible opening agent for the neural vascular barrier according to claim 2 or 3, wherein the polypeptide ligand is cyclophilin A lacking peptidyl-prolyl cis-trans isomerase activity.

5. The reversible opening agent for the neural vascular barrier according to any of claims 1 to 4, the agent being administered simultaneously or successively with at least one therapeutic agent selected from a therapeutic agent for neurodegenerative disease, a therapeutic agent for retinal disease, a therapeutic agent for psychiatric disorder, a therapeutic agent for central neural system tumor, and a therapeutic agent for epilepsy.
